# EUROPEAN PATENT APPLICATION

(11) **EP 1 356 784 A1**
(43) Date of publication of application: **29.10.2003**
(21) Application number: 02076676.2
(22) Date of filing: 25.04.2002
(51) Int. Cl.: A61F 2/04, A61F 6/20, C08F 220/18, C08F 226/10

(54) **Polymeric stent for vasovasostomy**

(71) Applicant: UroEnterprise B.V., 5582 KJ Waalre (NL)
(72) Inventor: Vrijhof, Henricus Josephus Elisabeth Johannes, 5582 KJ Waalre (NL)
(74) Representative: Prins, Adrianus Willem

(57) **Abstract**

Implantable tubular medical device for vasovasostomy in mammals and wherein the device is made from a hydrophilic bio-compatible polymer, the polymer comprising a hydrophilic component, preferably N-vinylpyrrolidone, a hydrophobic component, preferably N-butylacrylate and a cross-linker, preferably tetraethyleneglycol dimethacrylate and a method for the preparation of said polymer by polymerisation from its components.

## Description

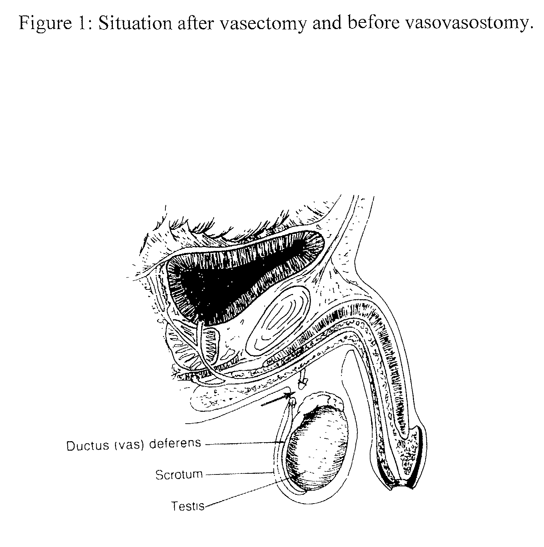

The invention relates to a new medical device which intends to facilitate and accelerate the urological operation which is known as vasovasostomy. Vasovasostomy is the reversal operation of vasectomy, which is normally performed as a method of birth control in males. Vasectomy is relatively simple, and has been performed on a routine basis since the 1920s. After administration of a local anaesthetic, a small incision is made in the scrotum, above one testicle. A small portion of the vas deferens is then removed (Figure 1), and the free ends are tied or cauterized to prevent their rejoining. The operation is carried out above both testicles.

Vasectomy produces some side-effects in the short term (*e*.*g*. some swelling and possibly also inflammation). Vasectomy is a popular and very effective method of birth control. It is reliable, cost-effective, maintenance free and essentially free of discomfort.

A microsurgical operation, called vasovasostomy, has been developed to reverse the vasectomy if, for some reason, the male has the desire to become fertile again. Vasovasostomy is successful in the large majority of cases, although a significant quantitative and qualitative reduction is noted in the semen, as compared to the original situation. Moreover, the semen quality can drop further on the long term, for example as a result of slow renarrowing of the vas deferens at the anastomotic site. There is consensus among urologists that the time that has passed between the original vasectomy and the vasovasostomy is an important indicator for the prediction of the success of vasovasostomy.

It is therefore a goal of the present invention to provide for means which allow for an efficient and quick procedure for vasovasostomy.

Accordingly, the invention relates to an implantable tubular medical device for vasovasostomy in mammals.

The device, also called a stent, facilitates the rejoining of the two loose ends of the vas deferens, in such way that renarrowing of the lumen of the vas deferens at the site of the anastomosis cannot occur. The stent is secured into position by at least three sutures, to prevent sperm leakage and exterior stent luxation. Each suture joins the testicular loose end of the vas deferens with the abdominal loose end of the vas deferens. In this way the stent is secured in its position. To implant such a device into a body, the device should be accepted by the body.

A preferred embodiment is therefore one wherein the device is made from biocompatible material. This also minimizes the chances for undesirable side-effects, such as inflammation, rejection, scar formation and/or blood-coagulation.

To facilitate the rejoining of the two loose end of the vas deferens by the stent, in such a way that renarrowing of the lumen of the vas deferens at the site of the anastomosis can not occur, a specific shape may provide these features.

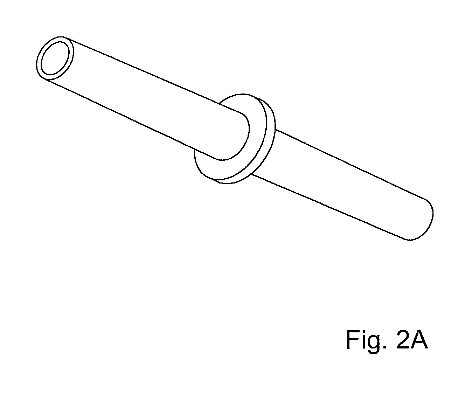

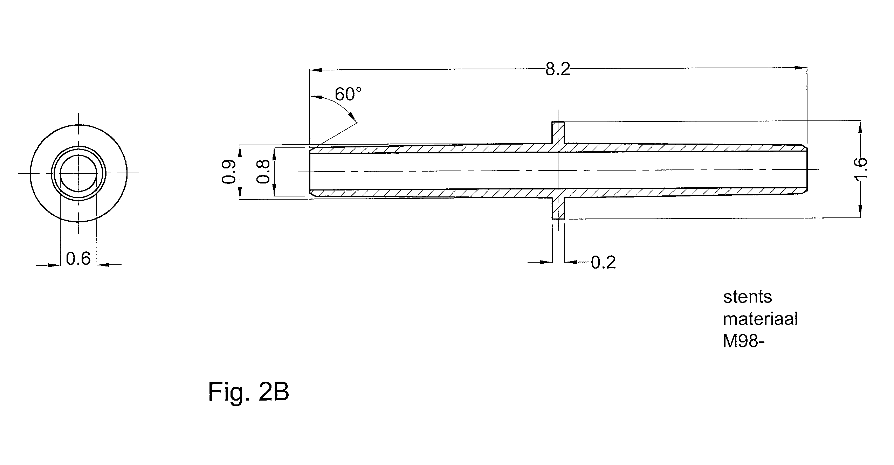

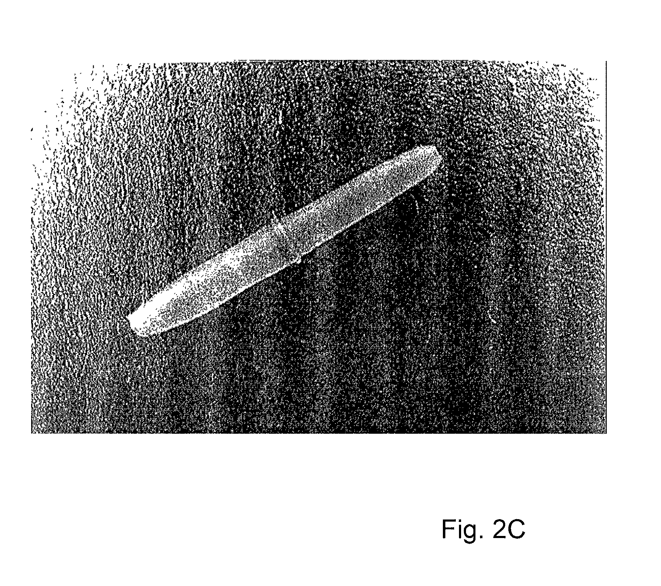

Accordingly, in an embodiment of the invention, the tubular form is conically shaped at both ends, as schematically indicated in Figure 2B. Suitable values for the angle α in Figure 2B range from 30 to 80°, preferably from 40 to 70°, *e*.*g*. 60°.

This holds the advantage that the stent can easily be inserted in the two loose ends of the vas deferens.

The stent may be provided with means to secure the position of the device. This is preferably in the form of a ridge, which can be positioned in the middle part of the device. The essential function of the ridge is to prevent the stent from migrating from the anastomosis area. In a preferred embodiment of the invention, the tubular form contains a ridge in the middle part of the device. Therefore any means by which the stent is kept in position are an embodiment of the invention.

The urologist uses three sutures to secure the stent into its position. Each suture joints the testicular loose end of the vas deferens, and the abdominal loose end of the vas deferens. In this way, the stent is secured into its position.

In order to secure the stent in its position, it is preferable that the biocompatible material from which the stent is made has certain characteristics. To fix the stent into its place, it is advantageous if the material has a certain stiffness for inserting in the vas deferens whereas after inserting and suturing, a certain accommodation of the stent is desired in respect to the characteristics of the tissue from the vas deferens. The material according to the invention is therefore designed such that it swells by absorption of water after its implantation. The stent construction then adopts mechanical characteristics which are comparable to the original vas deferens and the surrounding tissues.

In principle, the vasovasostomy stent can be manufactured from any suitable polymeric material. This material may have similar or slightly different swelling characteristics. The use of a polymeric material wherein the hydrophilic component is less dominant may result in a stent that can be handled over a larger time-window, thus facilitating an easier procedure for vasovasostomy. However, variations in the composition of the polymeric material, for instance in order to provide different swelling characteristics do not detract from the biocompatibility of the polymeric material.

By choosing the polymeric composition, the time-frame in which swelling occurs can be varied. If the operation can be performed quickly, swelling of the stent occurring within a time-frame of 1-2 minutes or longer is suitable.

Accordingly, in a preferred embodiment of the implantable medical device the biocompatible material provides stiffness in the dry state and provides rubbery mechanical properties in the wet state.

Another goal of this invention is the material from which the stent is constructed. The invention therefore also comprises the bio-material and the synthesis of the biomaterial out of which the stent is constructed.

The invention thus comprises a hydrophilic biocompatible material comprising a hydrophilic component and a hydrophobic component, wherein the molar ratio of the hydrophilic component and the hydrophobic component is from 0.1 to 10, preferably from 0.3 to 5, more preferably from 0.5 to 3.

In an embodiment of the invention the biocompatible material is further comprising up to 80 wt. % of a cross-linker.

The hydrophilic component of the biocompatible material is thought to provide the rubbery characteristics of the material after insertion into the vas deferens. Without wishing to be bound by any theory, it is thought that the swelling of the biocompatible material is caused by the absorption of water by the material. This is also a criterion for the selection of the hydrophilic component of the biocompatible material.

In an embodiment of the invention, the hydrophilic material is selected from methacrylates with hydrophilic side-chains such as 2-hydroxyethyl methacrylate and 2-N,N,-dimethylaminoethyl methacrylate or compounds such as N-vinylpyrrolidone. In a preferred embodiment the hydrophilic material is N-vinylpyrrolidone.

In an embodiment of the invention, the hydrophobic material is selected from hydrophobic acrylates and methacrylates, preferably N-butylmethacrylate.

The biocompatible material further comprises a cross-linking agent which is capable of cross-linking the hydrophilic and hydrophilic components.

In an embodiment of the invention the cross-linking agent is selected from the group of bifunctional reactive monomers of the methacrylate type, with hydrophilic characteristics, preferably tetraethylene glycol dimethacrylate.

The reactive monomers, the hydrophilic and hydrophobic components together with the cross-linker, are subjected to a polymerisation reaction. The invention therefore also relates to a method for the preparation of a hydrophilic biocompatible material comprising the polymerisation of a hydrophilic component and a hydrophobic component together with a cross-linker.

After polymerisation the resulting material in a preferred embodiment of the invention is a hydrophilic three-dimensional polymeric network, which is composed of N-vinylpyrrolidone as the hydrophilic constituent. Other constituents are N-butylmethacrylate (hydrophobic part), and the bifunctional crosslinker molecule tetraethyleneglycol dimethacrylate.

The biocompatible material can be prepared in homogeneous glass-like rods. The polymeric stents can be machined out of these rods, provided that the material is kept free of water. A computer-controlled lathe/mill instrument can be used in the manufacture of the stents but other techniques for the shaping of this material can also be used.

The stents have been tested in a rabbit vasovasostomy model.

The details of the operation are described in Example 2.

### Description of the figures:

Figure 1: shows the situation after vasectomy and before vasovasostomy where the two end of the vas deferens can be seen.
Figure 2A: Shows a representation of the vasovasostomy stent according to the present invention.
Figure 2B: Shows a schematic representation of the stent of Fig. 2A, including the measures in mm.
Figure 2C: Shows a picture of the stent according to the present invention as seen through a microscope (magnification 9 × ; the length of this stent is 9 mm). The material of this stent is obtained from N-vinylpyrrolidone and N-butylmethacrylate, using tetraethyleneglycol dimethacrylate as a bifunctional crosslinking agent.
Figure 3A: Transversal microscopic coupe of the vasovasostomy stent which was explanted from rabbit #3, 15 weeks post implantation.
Figure 3B: Transversal couple of the same vas deferens, approximately 1 cm proximal to the stent.

### Example 1.

### Preparation of the hydrophilic and biocompatible vasovasostomy stent material.

The construction material for the vasovasostomy stent was prepared from N-vinylpyrrolidone and N-butylmethacrylate, and the bifunctional crosslinking agent tetraethyleneglycol dimethacrylate. These reactive substances were polymerised suing AIBN as the radical initiator.

Typically, a mixture of N-vinylpyrrolidone 25.83 g, 232 mmol), n-butyl-methacrylate (14.17 g, 355 mmol), and tetraethyleneglycol dimethacrylate (220 mg, 0.2 mol %), and AIBN (24 mg, 0.044 mol %) were mixed and transferred into Teflon tubes (length: 20 cm, inner diameter: 8 mm, outer diameter: 10 mm). These tubes were tightly closed with a glass stopper on one end. The tubes were placed in a thermostated oil bath, which was interfaced with a time/temperature control system (PM Lauda, Köningshofen Germany). Subsequently, the following time/temperature profile was run: (i) first 60 min.: raise temperature from ambient to 60 °C; (ii), next 300 min.: temperature constant at 60 °C; (iii), next 60 min.: raise temperature to 80 °C; (iv), next 240 min.: temperature constant at 80 °C; (v), next 60 min.: raise temperature to 100 °C; (vi), next 240 min.: temperature constant at 100 °C; (vii), cool down to ambient temperature (unforced). This procedure afforded the desired material as hard transparent glassy rods, after careful opening the Teflon tubes. The lower and upper parts of the rods were cut off and discarded. The vasovasostomy stents were machined out of the remaining material.

### Example 2.

### Use of the vasovasostomy stent in the reconstruction of the vas deferens in a rabbit model study.

Twenty-six New Zealand white rabbits were used in a study to evaluate the performance of the vasovasostomy stent. The vas deferens of the animals (left and right) were cut and rejoined, either via the stent (n =13) or via single-layer microsurgical end-to-end anastomoses (n = 13).

Sperm cell counts revealed the functionality of the stent. Moreover, it was established that the operation is significantly accelerated and simplified. The results of the semen analyses are compiled in Tables 1 and 2.

**Table 1.**

| Sperm cell concentrations measured for the rabbits with two implanted mini-stents | | | | |
|---|---|---|---|---|
| Number | Sperm cells per mL, preoperative (millions) | Postoperative semen analysis at weeks | Mean number of sperm cells per mL, post-operative (millions) | Final number of sperm cells per mL (millions) |
| Mini-Stent Group | | | | |
| 1 | 160 | 4, 6, 9, 12 | 136 | 209 |
| 2 | 107 | 2, 4, 10, 13 | 403 | 710 |
| 3 | 1400 | 8, 12, 16 | 544 | 871 |
| 4 | 316 | 7, 13, 25, 37, 42 | 776 | 770 |
| 5 | 261 | 7, 17, 31, 36, 41 | 454 | 520 |
| 6 | 205 | 7, 17, 17, 31, 36, 41 | 540 | 1268 |
| 7 | 300 | 14, 31, 33, 38, 41 | 439 | 375 |
| 8 | 54 | 7, 22, 28, 31, 33 | 361 | 423 |
| 9 | 755 | 17, 24, 27, 28 | 663 | 425 |
| 10 | 845 | 17, 24, 27, 29 | 505 | 690 |
| 11 | 193 | 13, 23, 26, 28 | 395 | 371 |
| 12 | 125 | 13, 23, 26, 28 | 327 | 187 |
| 13 | 980 | 11, 21, 25, 26 | 569 | 76 |

**Table 2.**

| Sperm cell concentrations measured for the rabbits in the control group, on which conventional end-to-end vasovasostomy was performed | | | | |
|---|---|---|---|---|
| Number | Sperm cells per mL, preoperative (millions) | Postoperative semen analysis at weeks | Mean number of sperm cells per mL, post-operative (millions) | Final number of sperm cells per mL (millions) |
| End-to-end Group | | | | |
| 1 | 850 | 8, 14, 26, 38, 42 | 410 | 651 |
| 2 | 760 | 8, 14, 26, 38, 42, 4 | 300 | 154 |
| 3 | 324 | 7, 13, 25, 37, 41 | 312 | 233 |
| 4 | 785 | 7, 13, 26, 37, 42 | 650 | 960 |
| 5 | 414 | 7, 23, 37, 42, 47 | 531 | 258 |
| 6 | 625 | 7, 13, 25, 37 | 570 | 463 |
| 7 | 766 | 13, 30, 37, 40 | 437 | 450 |
| 8 | 249 | 13, 31, 37, 40 | 380 | 439 |
| 9 | 889 | 16, 23, 26, 28 | 421 | 620 |
| 10 | 324 | 16, 23, 26, 28 | 641 | 520 |
| 11 | 477 | 15, 22, 26, 29, 30 | 440 | 1267 |
| 12 | 283 | 15, 25, 28, 30 | 270 | 215 |
| 13 | 325 | 15, 22, 28, 40 | 492 | 480 |

From the data in Tables 1 and 2 it clearly follows that the postoperative sperm cell concentration of the animals which were operated using the stent of the present invention is improved as compared to the control group.

Furthermore, a more efficient use of labour and hospital facilities can be achieved through application of the stent. This is a significant conclusion, since microsurgical vasovasostomy has been identified as the treatment of choice for obstructive azoospermia as a result of vasectomy. This underscores the importance of the present invention.

Histological investigations were performed 14-15 weeks after the operation. The vas deferens of rabbit numbers 1, 2 and 3 containing a vasovasostomy stent was fixed in formaline. Numerous transversal sections were prepared and examined by light microscopy. In none of the three rabbits any tissue reaction of the vas deferens, surrounding the vasovasostomy stent was observed. The lumina of the vasovasostomy stents were open over their entire lengths, facilitating spermatozoa to pass the anastomosis.

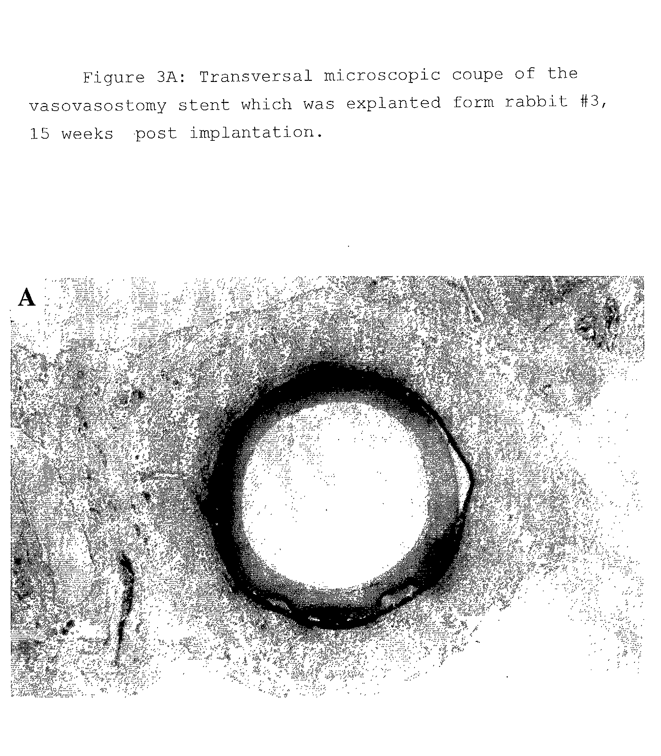

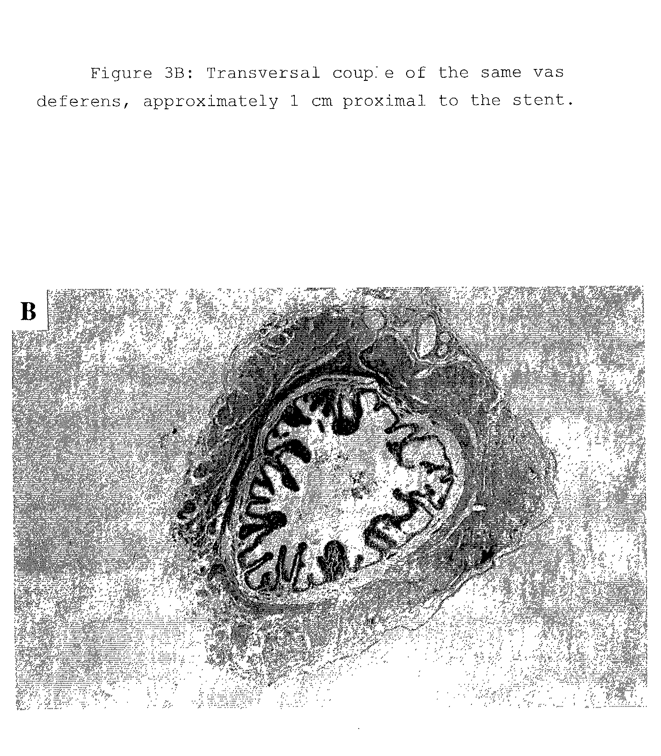

Figure 3 shows a representative histological transversal microscopic coupe of the vasovasostomy stent which was explanted from rabbit #3, 15 weeks post implantation (hematoxylin/eosin stain) (Figure 3A). Figure 3B shows a transversal coupe of the same vas deferens, proximal to the stent.

From the figures it can be clearly concluded that the stent according to the invention is suitable for the performance of vasovasostomy.

This pilot study clearly shows the efficacy of this vasovasostomy stent. It is possible to successfully perform the vasovasostomy on a rabbit model and the application of the stent leads to a patent stent lumen whereas the bio-material of the stent causes no adverse effects. Also, no strictures were seen in the stent group compared to the conventionally operated rabbits.

The medical device is functional in maintaining patency of the vas deference by exerting mechanical support to the vas deferens at the anatomotic site. Furthermore, the vasovasostomy stent substantially facilitates and accelerates the vasovasostomy procedure.

In an alternative embodiment of the present invention, the stent is shaped as a closed plug, rather than as a hollow tubular device. The outer shape and materials can be the same as described hereinabove. Since this plug according to this embodiment is not hollow, it may be used to close the testicular loose end of the vas deferens after it has been cut. In this way, the vas deferens is sealed and an effective sterilization is obtained as an alternative to conventional vasectomy. An advantage of this sterilization operation is that the reverse operation can be effected relatively easily by replacing the plug by the hollow stent described hereinabove.

## Claims

1. Implantable tubular medical device for vasovasostomy in mammals.

2. Implantable tubular medical device according to claim 1, wherein the device is made from biocompatible material.

3. Implantable tubular medical device according to claims 1 or 2, wherein the tubular form is conically shaped at both ends.

4. Implantable tubular medical device according to claims 1-3, wherein the tubular form contains a ridge in the middle part of the device.

5. Implantable medical device according to claim 1-4, wherein the biocompatible material provides stiffness in the dry state and provides rubbery mechanical properties in the wet state.

6. Hydrophilic biocompatible material comprising a hydrophilic component and a hydrophobic component, wherein the molar ratio of the hydrophilic component and the hydrophobic component is from 0.1 to 10, preferably from 0.3 to 5, more preferably from 0.5 to 3.

7. Hydrophilic biocompatible material according to claim 6 further comprising up to 80 wt.% of a cross-linker.

8. Material according to claims 6-7, wherein the hydrophilic material is N-vinylpyrrolidone.

9. Material according to claims 6-8, wherein the hydrophobic material is N-butylmethacrylate.

10. Material according to claims 6-9, wherein the cross-linking agent is tetraethyleneglycol dimethacrylate.

11. Method for the preparation of a hydrophilic biocompatible material according to claim 6, comprising the polymerisation of a hydrophilic component and a hydrophobic component together with a cross-linker.

12. Hydrophilic biocompatible material suitable for application in a stent for vasovasostomy.

13. Hydrophilic biocompatible material according to any of the claims 6-10 for application as a stent in vasovasostomy.

14. Use of hydrophilic biocompatible material as a stent in vasovasostomy.

15. Use of a device according to claims 1-5 for connecting biological channels in mammals.

16. Implantable non-hollow medical device, made from a biocompatible material having the form as defined in claims 3-5 and/or wherein the material is preferably the material defined in claims 6-10.

17. Use of a device according to claim 16 for sealing cut ends of biological channels in mammals.
